(19) Europäisches Patentamt
European Patent Office
Office européen des brevets

(11) **EP 1 921 143 A2**

(12) **DEMANDE DE BREVET EUROPEEN**

(43) Date de publication:
**14.05.2008 Bulletin 2008/20**

(21) Numéro de dépôt: **07076009.5**

(22) Date de dépôt: **10.04.2002**

(51) Int Cl.:
*C12N 15/10* (2006.01)    *C12N 15/01* (2006.01)
*C12N 15/54* (2006.01)    *C12N 15/70* (2006.01)
*C12N 9/12* (2006.01)     *C12N 1/21* (2006.01)
*C12R 1/19* (2006.01)     *C12R 1/19* (2006.01)

(84) Etats contractants désignés:
**AT BE CH CY DE DK ES FI FR GB GR IE IT LI LU MC NL PT SE TR**

(30) Priorité: **10.04.2001 FR 0104856**

(62) Numéro(s) de document de la (des) demande(s) initiale(s) en application de l'article 76 CBE:
**02761933.7 / 1 383 890**

(71) Demandeurs:
• **INSTITUT PASTEUR**
**75015 Paris (FR)**
• **CENTRE NATIONAL DE LA RECHERCHE SCIENTIFIQUE (CNRS)**
**75016 Paris (FR)**

(72) Inventeurs:
• **Marliere, Philippe**
**75012 Paris (FR)**
• **Pochet, Sylvie**
**75012 Paris (FR)**
• **Bouzon, Madeleine**
**92190 Meudon (FR)**

(74) Mandataire: **Caen, Thierry Alain Santarelli**
**14 avenue de la Grande Armée**
**B.P. 237**
**75822 Paris Cedex 17 (FR)**

Remarques:
Cette demande a été déposée le 21-11-2007 comme demande divisionnaire de la demande mentionnée sous le code INID 62.

(54) **Mutants de la désoxycytidine kinase possédant une activité enzymatique élargie**

(57) La présente invention se rapporte à un procédé d'évolution artificielle *in vivo* de protéines, ledit procédé permettant de faire évoluer une protéine X par complémentation d'une protéine apparentée Y, X et Y appartenant tous deux à la même classe de la nomenclature enzymatique EC ou à des classes voisines. Les mutants D133E et R104Q de la désoxycytidine kinase (DCK) ont été obtenus, chacune de ces mutations conférant l'acquisition de l'activité thymidine kinase par DCK.

EP 1 921 143 A2

**Description**

La présente invention se rapporte à un procédé d'évolution artificielle *in vivo* de protéines, ledit procédé permettant de faire évoluer une protéine X par complémentation d'une protéine apparentée Y, X et Y appartenant tous deux à la même classe de la nomenclature enzymatique EC ou à des classes voisines. Les mutants D133E et R104Q de la désoxycytidine kinase (DCK) ont été obtenus, chacune de ces mutations conférant l'acquisition de l'activité thymidine kinase par DCK.

Les techniques de séquençage et d'amplification PCR font appel à une gamme de plus en plus diversifiée de nucléosides triphosphates, les monomères activés qui peuvent être condensés par les ADN polymérases. De tels monomères artificiels se distinguent des quatre monomères naturels tant par des altérations chimiques de la base hétérocyclique [Sala et al, 1996] que du sucre pentose et du groupement triphosphate. La préparation des dérivés triphosphate s'effectue généralement à partir des nucléosides correspondants en soumettant l'alcool 5' libre à une phosphorylation puis en condensant le 5' phosphate au pyrophosphate, pour donner le triphosphate. La catalyse de l'étape de phosphorylation par une nucléoside kinase consommant de l'ATP constitue un procédé valide à l'échelle industrielle. Un tel procédé de synthèse n'est envisageable qu'avec des enzymes présentant une activité élargie, c'est à dire des nucléosides kinases capables de phosphoryler n'importe quel nucléoside avec une efficacité similaire. D'une manière plus générale, l'obtention d'enzymes présentant des activités élargies permettrait d'avoir accès à des outils puissants pour toute sorte d'applications biotechnologiques.

Diverses solutions pour effectuer des mutations dirigées dans une molécule d'ADN ont été décrites dans l'état de la technique. Ces techniques consistent à introduire *in vitro* une mutation, une délétion ou une insertion en un site déterminé dans une molécule d'ADN par exemple en utilisant la PCR. Ces diverses techniques sont décrites dans Hall, et al. Protein Eng. 4:601 (1991); Hemsley, et al. Nucleic Acids Research 17:6545-6551 (1989); Ho, et al. Gene 77:51-59 (1989); Hultman, et al. Nucleic Acids Research 18:5107-5112 (1990); Jones, et al. Nature 344:793-794 (1990); Jones, et al. Biotechniques 12:528-533 (1992); Landt, et al. Gene 96:125-128 (1990); Nassal, et al. Nucleic Acids Research 18:3077-3078 (1990); Nelson, et al. Analytical Biochemistry 180:147-151 (1989); Vallette, et al. Nucleic Acids Research 17:723-733 (1989); Watkins, et al. Biotechniques 15:700-704 (1993); Weiner, et al. Gene 126:35-41 (1993). Yao, et al. PCR Methods and Applications 1:205-207 (1992) et dans Weiner et al, Gene 151:1/9-123(1994).
Outre les problèmes techniques rencontrés, il est impossible de savoir à l'avance quelle serait l'effet d'une mutation donnée sur l'activité d'une protéine avec de telles techniques.

D'autres méthodes consistent à introduire des mutations au hasard dans le génome par l'emploi d'agents mutagènes (2-aminopurine, hydroxylamine ou ACRIDINE) et à sélectionner les cellules ou organismes montrant le phénotype recherché. Néanmoins, ces méthodes conduisent à l'introduction de nombreuses mutations, parfois létales, et ne sont pas adaptées à faire évoluer une protéine donnée dans un but précis.
L'art antérieur montre également que l'on peut employer des systèmes *in vivo,* par exemple en utilisant des ADN polymérase exo-, ou d'autres protéines pouvant introduire des mutations (US 6,015,705), mais aucune de ces techniques ne s'apparente à la méthode proposée par la présente invention.

Pour répondre aux besoins et problèmes évoqués précédemment, la présente invention propose un procédé d'évolution artificielle *in vivo* de protéines. Ce procédé permet de faire évoluer *in vivo* une protéine X par complémentation d'une protéine apparentée Y, X et Y appartenant tous deux à la même classe de la nomenclature enzymatique EC ou à des classes voisines. En effet, on a démontré qu'il est possible de modifier par mutation l'activité d'un enzyme d'une classe, non seulement à l'intérieur d'une même classe, mais également de lui faire acquérir les activités caractérisant les classes voisines (3 premiers chiffres de la nomenclature EC en commun). En d'autres termes, l'invention apporte un procédé pour faire évoluer une protéine X pour qu'elle acquière l'activité d'une autre protéine Y, X conservant au moins une de ses propriétés ou activités initiales et donc présentant *in fine* une activité élargie.

Ce procédé est particulièrement adapté à des enzymes du type nucléotidyl kinase, phosphorylase et nucléotidyl transférases, car on peut mettre à profit leur capacité à introduire des mutations dans leur propre gène.
A ce titre, l'invention a été mise en oeuvre pour la désoxycytidine kinase d'Homo sapiens (DCK) qui est un enzyme capable de phosphoryler une large gamme de nucléosides présentant une parenté chimique avec la désoxycytidine (dC), suivant la réaction :

$$dC + ATP \rightarrow dCMP + ADP.$$

En plus de la désoxycytidine, cet enzyme reconnaît comme substrats les désoxynucléosides puriques dA et dG ainsi que des analogues structuraux de bases ou de sucres, comme la 5-aza-cytidine (5azadC) et l'arabinocytidine. Toutefois, la thymidine n'est pas substrat de l'enzyme *in vitro* [Datta et al, 1989]. L'ADNc spécifiant la désoxycytidine kinase humaine a été cloné et séquencé [Chottiner et al, 1991, numéro d'accession dans GENBANK : M60527], révélant des similarités entre DCK et les thymidine kinases des virus herpétiques [Harrison et al, 1991]. De même, la structure tridimensionnelle de la thymidine kinase de l'herpès a été résolue par radiocristallographie [Brown et al, 1995]. Le brevet américain US 6,063,376 décrit une seconde désoxycytidine kinase humaine appelée DCK2 qui possède 60 % d'identité avec DCK1.

Dans le procédé selon l'invention, on a mis à profit la propriété de mutateur conditionnel de DCK en présence d'analogues de nucléosides promutagènes pour soumettre son propre gène dck à un épisode de mutagénèse in vivo.

Ainsi, des bactéries de génotype Δ*deo tdk p::dckH*+ furent exposées soit à la 2-amino-2'-désoxyribosyl-purine (disoA) soit à la 2-amino-désoxyribosyl-2-hydroxy-purine (disoG), puis incubées sur milieu solide riche en présence de triméthoprime et de thymidine. Des colonies sont apparues suite à l'administration des deux composés à une fréquence de l'ordre de $10^{-8}$. Aucune colonie ne survint en absence de nucléoside promutagène.

Le séquençage des gènes de 7 plasmides mutants obtenus indépendamment (4 après mutagénèse par disoG, 3 par disoA) a mis en évidence deux mutations ponctuelles D133E et R104Q, chacune conférant l'acquisition de l'activité thymidine kinase par DCK. En outre, un plasmide réunissant les deux mutations dans un même allèle a été construit et introduit dans la souche β7117 de génotype Δ*deo tdk.* Il a permis la complémentation de la tdk inactivée par mutation ou délétion, exprimant donc aussi une activité thymidine kinase.

## Description

Ainsi, de manière générale, la présente invention se rapporte à un procédé d'évolution *in vivo* artificielle de protéines, ledit procédé permettant de faire évoluer *in vivo* une protéine X par complémentation d'une protéine apparentée Y, X et Y appartenant tous deux à la même classe de la nomenclature enzymatique EC ou à des classes voisines.

Un tel procédé permet de faire évoluer une protéine X de sorte à en modifier ses caractéristiques et comprend les étapes suivantes :

a) obtention de cellules comportant un génotype [protéine Y∗ : :protéine X+], par transformation de cellules [protéine Y∗] avec un acide nucléique comprenant le gène codant pour la protéine X, Y∗ signifiant que le gène codant pour Y a été inactivé, Y étant une protéine appartenant à une classe voisine de X possédant une activité voisine, les classes de X et Y se caractérisant en ce qu'elles possèdent au moins les trois premiers chiffres appartenant aux classes EC de la nomenclature internationale à 4 chiffres, lesdites cellules présentant un phénotype auxotrophe nécessitant pour survivre l'apport du produit de la réaction de Y sur son substrat dans le milieu de culture ;
b) exposer les cellules obtenues à l'étape a) à un mutagène,
c) mettre en culture lesdites cellules dans un milieu comprenant le substrat de Y, le produit de la réaction de Y sur son substrat étant nécessaire à la survie de ladite cellule,
d) sélectionner les cellules qui ont survécu à l'étape c) dans lesquelles la protéine X, modifiée par l'action dudit agent mutagène, complémente la déficience en la protéine Y.

A l'étape b), toute technique visant à augmenter la sensibilité de la cellule vis-à-vis d'un mutagène ou d'un promutagène, par exemple par expression d'une kinase, d'une phosphorylase ou d'une ADN pol exo-, peut être utilisée. De préférence, on utilise un vecteur d'expression de la DCK1 comportant les mutations D133E et R104Q décrite ci-après.

Dans le cadre de l'invention, le terme «Y∗» signifie que le gène codant pour Y a été inactivé, c'est à dire qu'il a été délété en tout ou en partie, ou inactivé par insertion d'une séquence ou par introduction d'une mutation. Il faut signaler que l'invention peut également être exécutée dans le cas de modification du gène Y conduisant à un phénotype du type Ts (température sensible). Dans ce cas, les cellules sont cultivées à des températures non permissibles pendant la phase de sélection (étapes c) et d)).

Parmi, les protéines que l'on cherche à faire évoluer, on peut citer notamment :
- les protéines appartenant à la famille des kinases, telles que par exemple

| Numéro EC | Nom selon la nomenclature internationale |
|---|---|
| 2.7.1.20 | Adenosine kinase. |
| 2.7.1.21 | Thymidine kinase. |
| 2.7.1.38 | Phosphorylase kinase. |
| 2.7.1.49 | Hydroxymethylpyrimidine kinase. |
| 2.7.1.74 | désoxycytidine kinase (DCK). |
| 2.7.4.6 | Nucleoside-diphosphate kinase. |
| 2.7.4.7 | Phosphomethylpyrimidine kinase. |
| 2.7.4.8 | Guanylate kinase. |
| 2.7.4.9 | Thymidylate kinase. |
| 2.7.4.10 | Nucleoside-triphosphate--adenylate kinase. |

(suite)

| Numéro EC | Nom selon la nomenclature internationale |
|---|---|
| 2.7.4.11 | (Deoxy)adenylate kinase. |
| 2.7.4.12 | T2-induced deoxynucleotide kinase. |
| 2.7.4.13 | (Deoxy)nucleoside-phosphate kinase. |

- les nucléotidyl transférase, telles que par exemple

| Numéro EC | Nom selon la nomenclature internationale |
|---|---|
| 2.7.7.6 | DNA-directed RNA polymerase. |
| 2.7.7.7 | DNA-directed DNA polymerase. |
| 2.7.7.8 | Polyribonucleotide nucleotidyltransferase. |
| 2.7.7.19 | Polynucleotide adenylyltransferase. |
| 2.7.7.25 | tRNA adenylyltransferase. |
| 2.7.7.48 | RNA-directed RNA polymerase. |
| 2.7.7.49 | RNA-directed DNA polymerase. |
| 2.7.7.50 | mRNA guanylyltransferase. |

- les phosphorylases, telles que par exemple

| Numéro EC | Nom selon la nomenclature internationale |
|---|---|
| 2.4.2.1 | Purine-nucleoside phosphorylase. |
| 2.4.2.2 | Pyrimidine-nucleoside phosphorylase. |
| 2.4.2.3 | Uridine phosphorylase. |
| 2.4.2.4 | Thymidine phosphorylase. |
| 2.4.2.7 | Adenine phosphoribosyltransferase. |
| 2.4.2.8 | Hypoxanthine phosphoribosyltransferase. |
| 2.4.2.9 | Uracil phosphoribosyltransferase. |
| 2.4.2.15 | Guanosine phosphorylase. |
| 2.4.2.23 | Deoxyuridine phosphorylase. |
| 2.4.2.28 | 5'-methylthioadenosine phosphorylase. |

Bien entendu, d'autres enzymes, notamment les enzymes du métabolisme ou du catabolisme, peuvent faire l'objet d'une évolution grâce au procédé selon l'invention. Ces enzymes et leur numéro EC respectif sont répertoriés par le Committee of the International Union of Biochemistry and Molecular Biology (IUBMB) à l'adresse suivante : http://expasy.proteome.org.au/enzyme/

Dans un mode de réalisation préféré, l'invention se rapporte à un procédé tel que défini ci-dessus dans lequel la protéine X a la propriété d'introduire des mutations dans l'ADN. La propriété de mutateur conditionnel de la protéine X en présence d'analogues de nucléosides promutagènes permet de soumettre son propre gène à un épisode de mutagénèse *in vivo.*

En ce sens, le procédé selon l'invention permet de faire évoluer une kinase X de sorte à en modifier ses caractéristiques, ledit procédé comprenant les étapes suivantes :

a) obtention de cellules comportant un génotype [kinaseY∗ :kinaseX+], par transformation d'une cellule [kinaseY∗] avec un acide nucléique comprenant le gène codant pour la kinase X, Y∗ signifiant que le gène codant pour Y a été inactivé, Y étant une kinase appartenant à une classe voisine de X montrant une activité voisine, les classes de X et Y se caractérisant en ce qu'elles possèdent au moins les trois premiers chiffres appartenant aux classes EC 2.7.1.- de la nomenclature internationale à 4 chiffres, lesdites cellules présentant un phénotype auxotrophe nécessitant pour survivre l'apport du produit de la réaction de Y sur son substrat dans le milieu de culture ;

b) exposer les cellules obtenues à l'étape a) à un analogue de nucléosides promutagènes pendant un temps donné, la kinase X étant capable de phosphoryler ledit analogue,

c) mettre en culture lesdites cellules dans un milieu comprenant le substrat de Y, le produit de la réaction dudit substrat avec Y étant nécessaire à la survie de ladite cellule,

d) sélectionner les cellules qui ont survécu à l'étape c) dans lesquelles la kinase X, modifiée par l'action dudit analogue de nucléoside promutagène, complémente la déficience en la kinase Y.

Par « complément », on entend la suppression du phénotype auxotrophe résultant de l'inactivation du gène Y.

Lesdites cellules sont des cellules procaryotes ou eucaryotes, de préférence *E. coli*. Dans le cas où la protéine X est une kinase, le substrat est sélectionné parmi les nucléosides et leurs analogues.

Avantageusement, la kinase X est une déoxycytidine kinase appartenant à la classe EC 2.7.1.74. La kinase Y est de préférence une kinase n'appartenant pas à EC 2.7.1.74, notamment une thymidine kinase (TDK) appartenant à la classe EC 2.7.1.21. Dans la mesure où X est une phosphorylase ou une polymérase, Y est une phosphorylase ou une polymérase différente de X.

Ainsi, le procédé évoqué ci-dessus peut comprendre les étapes suivantes :

a) obtention d'une bactérie *E. coli ∆deo tdk p::dckH+,* mise en culture des cellules obtenues à l'étape a) dans un milieu comprenant :

- un agent mutagène sélectionné parmi les analogues de nucléosides promutagènes et du triméthoprime qui bloque la synthèse du thymidylate par la thymidylate synthase;
- et la thymidine qui est nécessaire à la survie de ladite cellule,

b) sélection des cellules qui ont survécu à l'étape b) dans lesquelles la DCKH, modifiée par l'action dudit analogue promutagène, complémente la déficience en TDK.

Avantageusement, la kinase X est une désoxycytidine kinase, notamment la DCK1 humaine de séquence déposée dans GENBANK sous le numéro d'accession M60527 comportant au moins une mutation sélectionnée parmi les mutations D133E et R104Q. La séquence double mutante de DCK1 (SEQ ID No1) est :

```
  1    MATPPKRSCP   SFSASSEGTR   IKKISIEGNI   AAGKSTFVNI
       LKQLCEDWEV VPEPVARWCN

 61    VQSTQDEFEE   LTMSQKNGGN   VLQMMYEKPE   RWSFTFQTYA
       CLSRIRAQLA SLNGKLKDAE

121    KPVLFFERSV   YSDRYIFASN   LYESECMNET   EWTIYQDWHD
       WMNNQFGQSL ELDGIIYLQA

181    TPETCLHRIY   LRGRNEEQGI   PLEYLEKLHY   KHESWLLHRT
       LKTNFDYLQE VPILTLDVNE

241 DFKDKYESLV EKVKEFLSTL
```

En outre, la kinase X est de préférence capable d'activer un analogue de nucléoside promutagène, lequel analogue introduit des mutations dans son propre gène. A l'issu du procédé, la kinase X mutée est capable de remplacer (complémentation) la kinase Y et présente donc une activité élargie par rapport à son activité initiale.

Ainsi, dans un deuxième aspect, l'invention vise une protéine X mutée, notamment une kinase X mutée, susceptible d'être obtenue à partir du procédé décrit ci-dessus, caractérisée en ce qu'elle présente une activité élargie par rapport à la protéine X initiale (ou à la kinase X initiale). Il peut s'agir par exemple de la kinase DCK1 humaine mentionnée ci-dessus comportant au moins une mutation sélectionnée parmi les mutations D133E et R104Q. L'invention porte également sur un acide nucléique comprenant une séquence codant pour la kinase DCK1 humaine telle que définie ci-dessus et un vecteur comprenant cette séquence codante, ladite séquence pouvant être fusionnée à un promoteur efficace dans les cellules eucaryotes et/ou procaryotes.

De préférence, le vecteur est un plasmide qui peut être introduit dans une bactérie telle que par exemple *E.*

*coli* par transformation ; le vecteur se maintient dans la bactérie de manière stable ou transitoire.

L'invention porte également sur une cellule hôte comprenant un vecteur exposé ci-dessus.

Un autre aspect de l'invention concerne l'utilisation d'une kinase, explicitée précédemment, dans un procédé tel que défini ci-dessus pour rendre actif un analogue de nucléoside promutagène. Plus généralement, l'invention vise l'utilisation d'une kinase mentionnée ci-dessus dans tout procédé d'hypermutagénèse, pour convertir des nucléosides, naturellement réfractaires à la phosphorylation enzymatique, en leur dérivé 5' phosphate respectif.

L'invention porte également sur l'utilisation concomitante d'une kinase selon l'une des revendications 12 et 13 et d'autres enzymes tels que AMK et/ou la transdesoxyribosylase (NTD) pour élargir in *vivo* la gamme de la mutagénèse à l'aide de promutagènes.

En outre, il faut signaler que le vecteur mentionné ci-dessus peut être utilisé pour la préparation d'un médicament destiné à la thérapie génique pour permettre l'incorporation d'analogues de nucléosides dans l'ADN.

L'invention vise également un procédé de mutagénèse *in vivo* d'une séquence d'ADN déterminée, ladite séquence d'ADN étant dans une cellule, comprenant les étapes consistant à :

- effectuer la mutation par insertion d'au moins un type de nucléosides promutagènes dans ladite séquence, la cellule exprimant au moins un système enzymatique permettant l'insertion dudit nucléotide promutagène dans l'ADN,
- et détecter la présence de la séquence mutée,

caractérisé en ce que le système enzymatique comprend une kinase telle que définie ci-dessus.

Ce procédé permet de faire évoluer une protéine spécifique de la cellule. En ce sens, on inactive un gène codant pour une protéine apparentée à ladite protéine spécifique, la protéine apparentée étant nécessaire à la survie de la cellule, et on détecte la complémentation après mutation du gène codant pour ladite protéine spécifique. Ladite protéine spécifique et ladite protéine apparentée sont de préférence des enzymes appartenant à une classe voisine ayant en commun au moins les trois premiers chiffres de la nomenclature internationale EC à 4 chiffres.

Ces protéines sont sélectionnées parmi les kinases appartenant aux classes EC 2.7.1. : on peut également envisager les nucléotidyl transférases appartenant aux classes EC 2.7.7. -notamment les polymérases, et les phosphorylases appartenant aux classes EC 2.4.2.- en utilisant des cribles appropriés. Avantageusement, lesdites protéines ont la propriété de pouvoir faire évoluer leur propre gène.

L'invention porte également sur la souche d'*E. coli* β 7151 de génotype Δ*deo tdk* comprenant un vecteur exprimant la DCK mutée D133E déposée le 21 février 2001 à la CNCM (Collection Nationale de Cultures de Microorganismes, 25, rue du Docteur Roux, 75724 Paris cedex 15, France) sous le numéro d'accession I-2631.

L'invention porte également sur une souche d'*E. coli* β 7134 de génotype Δ*deo tdk* comprenant un vecteur exprimant la DCK humaine déposée le 21 février 2001 à la CNCM sous le numéro d'accession I-2630.

L'invention porte également sur une souche d'*E. coli* β7338 de génotype Δ*deo tdk* comprenant un vecteur exprimant la DCK double mutante D133E et R104Q déposée le 27 mars 2001 à la CNCM sous le numéro d'accession I-2650.

**Légendes**

Figure 1: crible permettant la sélection d'une activité thymidine kinase chez *Escherichia coli.*

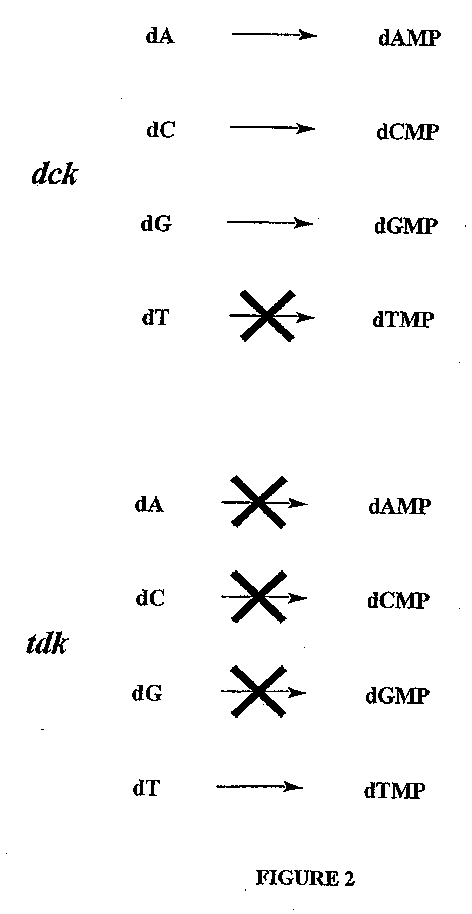

Figure 2: spécificités comparées de la thymidine kinase d'*Escherichia coli* et de la désoxycytidine kinase humaine.

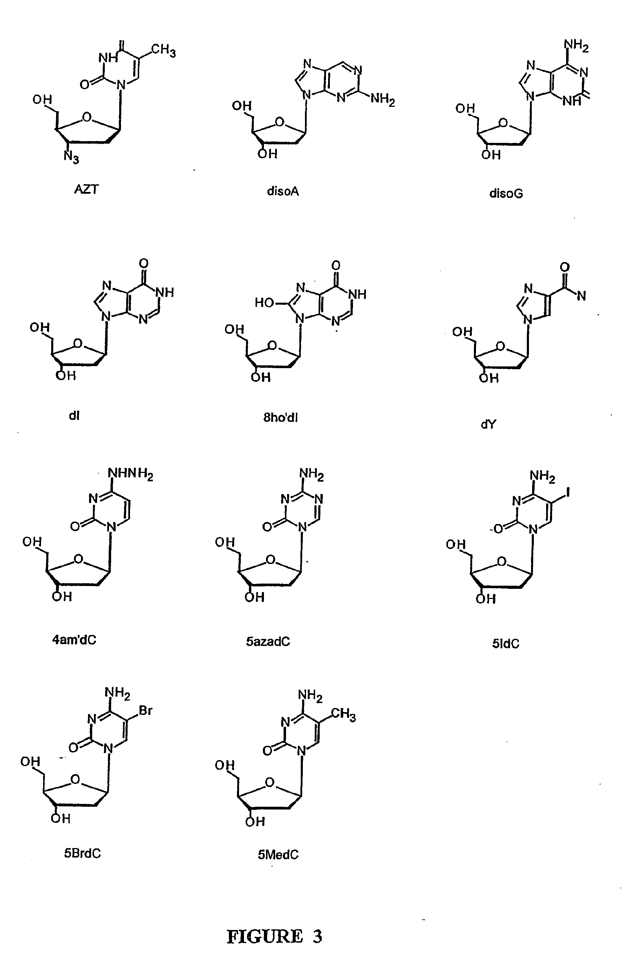

Figure 3: exemples d'analogues de nucléosides

**Matériels et méthodes**

**Composés chimiques :** les composés 3'-azido-3'-désoxythymidine (AZT), 2'-désoxyinosine (dI), N4-amino-2'-désoxycytidine (désoxyribonucléoside de la 2-hydroxy-4-hydrazino-pyrimidine, désigné 4am'dC), 5-aza-2'-désoxycytidine (5azadC), 5-iodo-2'-désoxycytidine (5IdC), 5-bromo-2'-désoxycytidine (5BrdC) and 5-methyl-2'-désoxycytidine (5MedC) ont été achetés chez Sigma. La 2-désoxy-iso-adénosine (disoA) (2-amino-9-(2'-désoxy-β-D-ribofuranosyl) purine) a été préparée par transglycosylation enzymatique en utilisant un extrait brut de N-deoxyribosyltransferases de *Lactobacillus leichmannii*. La synthèse de la 2'-désoxyisoguanosine (2-hydroxy-6-amino-9-(2'-deoxy-β-D-ribofuranosyl) purine) a été réalisée par fermeture du cycle du 5-amino-1-(2'-désoxy-β-D-ribofuranosyl)imidazole-4-carboxamide. La préparation du 1-(2'-désoxyribofuramosyl)-imidazole-4-carboxamide (dY) a été décrite dans Pochet et al, 1995.

**Culture des souches bactériennes** : les bactéries ont été cultivées dans un milieu riche Luria-Bertani (LB) ou dans un milieu minimum supplémenté avec 2g/l de glucose (MS). Les mêmes milieux de croissance ont été solidifiés avec 15g/l d'agar (Difco) pour la préparation des boîtes de Pétri. Les cultures liquides et solides ont été incubées à 37°C. Dans certains cas, des antibiotiques ont été rajoutés aux concentrations suivantes : 100mg/l de carbenicilline, 25mg/l de kanamycine, 15mg/l de tétracycline. Le triméthoprime a été utilisé à une concentration de 100 mg/l dans un milieu LB supplémenté avec 0,3 mM de thymidine. Pour l'induction de l'expression du gène, l'isopropyl-β-D-thiogalactoside (IPTG) a été rajouté à 0,5 mM.

**Souches et constructions plasmidiques :** la liste des souches *E. coli* K12 utilisées et les plasmides construits dans le cadre de la présente invention est donnée dans le tableau 1 ci-après.

**Tableau 1 : souches bactériennes et plasmides**

| Souche | Phénotype | Construction |
|---|---|---|
| MG1655 | F- λ- | B. Bachmann |
| KU8 | *trxB::Tn10Kan ΔserB* zjj::*Tn10* | Uhland et al. |
| SØ928 | *Δdeo Δlac thi upp udp ton* | P. Nygaard |
| SØ5110 | *cdd::Tn10* | P. Nygaard |
| CC101 | *ara Δ(lac proB)13* | Cupples and Miller |
|  | F' *lacZ:* Glu461am *proB*+ |  |
| CC102 | *ara Δ(lac proB)13* | Cupples and Miller |
|  | F' *lacZ*:Glu461Gly *proB*+ |  |
| CC103 | *ara Δ(lac proB)13* | Cupples and Miller |
|  | F' *lacZ:* Glu461Gln *proB*+ |  |
| CC104 | *ara Δ(lac proB)13* | Cupples and Miller |
|  | F' *lacZ*:Glu461Ala *proB*+ |  |
| CC105 | *ara Δ(lac proB)13* | Cupples and Miller |
|  | F' *lacZ:*.Glu461 Val *proB*+ |  |
| CC106 | *ara Δ(lac proB)13* | Cupples and Miller |
|  | F' *lacZ:*Glu461Lys *proB*+ |  |
| β7069 | *tdk* | Mutant MG1655 (résistance spontanée à l' AZT) |
| β7117 | *Δdeo tdk* | Transductions séquentielles de β7069 avec des lysats P1 de KU8 et SØ928 |
| β7134 | *Δdeo tdk* pDCK1 (*bla*+ *lacI*Q *dck*+) | Transformation de β7117 |
| β7151 | *Δdeo tdk* | Plasmide pDCK D133E |
| β7320 | *Δdeo tdk cdd::*Tv10 | Transduction de β7117 avec le lysat P1 de SØ5110 |
| β7334 | *Δdeo tdk cdd::*Tv10 pSUTrc (*kan*+ *lacI*Q) | Transformation de β7320 |
| β7335 | *Δdeo tdk cdd::*Tv10 pSUDCK1 (*kan*+ *lacI*Q dck+) | Transformation de β7320 |
| β7336 | *Δdeo tdk cdd::*Tv10 pSUDCK2 (*kan*+ *lacI*Q *dck:* D133E) | Transformation de β7320 |
| β7337 | *Δdeo tdk cdd::*Tv10 pSUDCK3 (*kan*+ *lacI*Q *dck: R104Q)* | Transformation de β7320 |
| β7338 | *Δdeo tdk cdd::*Tv10 pSUDCK4 (*kan*+ *lacI*Q *dck:*D133E-R104Q) | Transformation de β7320 |
| β7339 | *Δdeo tdk cdd::*Tv10 pSUTrc (*kan*+ *lacI*Q) pAK1 (*bla*+ *lacI*Q *adk*+) | Double transformation de β7320 |
| β7340 | *Δdeo tdk cdd*::*Tn10* pSUDCK1 *(kan*+ *lacI*Q *dck*+) pAK1 (*bla*+ *lacI*Q *adk*+) | Double transformation de β7320 |
| β7341 | *Δdeo tdk cdd*::*Tn10* pSUDCK2 (*kan*+ *lacI*Q *dck*:D133E) pAK1 (*bla*+ *lacI*Q *adk*+) | Transformation de β7336 |
| β7342 | *Δdeo tdk cdd::*Tv10 pSUDCK3 (*kan*+ *lacI*Q *dck:* R104Q) pAK1 (*bla*+ *lacI*Q *adk*+) | Double transformation de β7320 |

(suite)

| Souche | Phénotype | Construction |
|--------|-----------|--------------|
| β7343 | Δ*deo tdk cdd::*Tν10<br>pSUDCK4 (*kan⁺ lacIQ dck*:D133E-R104Q)<br>pAK1 (*bla⁺ lacIQ adk⁺*) | Double transformation de β7320 |
| β7344 | Δ*deo tdk cdd::*Tν10<br>pSUTrc (*kan⁺ lacIQ*)<br>pAKT39A (*bla⁺ lacIQ adk*:T39A) | Double transformation de β7320 |
| β7345 | Δ*deo tdk cdd::*Tν10<br>pSUDCK1 (*kan⁺ lacIQ dck⁺*)<br>pAKT39A (*bla⁺ lacIQ adk*:T39A) | Double transformation de β7320 |
| β7346 | Δ*deo tdk cdd::*Tν10<br>pSUDCK2 (*kan⁺ lacIQ dck*:D133E)<br>pAKT39A (*bla⁺ lacIQ adk*:T39A) | Double transformation de β7320 |
| β7347 | Δ*deo tdk cdd::*Tν10<br>pSUDCK3 (*kan⁺ lacIQ dck*:R104Q)<br>pAKT39A (*bla⁺ lacIQ adk*:T39A) | Double transformation de β7320 |
| β7348 | Δ*deo tdk cdd::*Tν10<br>pSUDCK4 (*kan⁺ lacIQ dck*:D133E-R104Q)<br>pAKT39A (*bla⁺ lacIQ adk*:T39A) | Double transformation de β7320 |
| β7351 | *ara* Δ(*lac proB*)*13*<br>F' *lacZ*:Glu461*am proB⁺*<br>pSUDCK2 (*kan⁺ lacIQ dck*:D133E) | Transformation de CC101 |
| β7352 | *ara* Δ(*lac proB*)*13*<br>F' *lacZ*:Glu461Gly *proB⁺*<br>pSUDCK2 (*kan⁺ lacIQ dck*: D133E) | Transformation de CC102 |
| β7353 | *ara* Δ(*lac proB*)*13*<br>F' *lacZ*:Glu461Gln *proB⁺*<br>pSUDCK2 (*kan⁺ lacIQ dck*:D133E) | Transformation de CC103 |
| β7354 | *ara* Δ(*lac proB*)*13*<br>F' *lacZ*:Glu461Ala *proB⁺*<br>pSUDCK2 (*kan⁺ lacIQ dck*: D133E) | Transformation de CC104 |
| β7355 | *ara* Δ(*lac proB*)13<br>F' *lacZ:* Glu461 Val *proB⁺*<br>pSUDCK2 (*kan⁺ lacIQ dck*:D133E) | Transformation de CC105 |
| β7356 | *ara* Δ(*lac proB*)*13*<br>F' *lacZ*:Glu461Lys *proB⁺*<br>pSUDCK2 (*kan⁺ lacIQ dck*:D133E) | Transformation de CC106 |
| β7357 | *ara* Δ(*lac proB*)*13*<br>F'*lacZ*:Glu461*am proB⁺*<br>pSUDCK2 (*kan⁺ lacIQ dck*:D133E)<br>pAK1 (*bla⁺ lacIQ adk⁺*) | Double transformation de CC101 |
| β7358 | *ara* Δ(*lac proB*)*13*<br>F'*lacZ*.-Glu461Gly/*proB⁺*<br>pSUDCK2 (*kan⁺ lacIQ dck*:D133E)<br>pAK1 (*bla⁺ lacIQ adk⁺*) | Double transformation de CC102 |
| β7359 | *ara* Δ(*lac proB*)*13*<br>F' *lacZ*:Glu461Gln *proB⁺*<br>pSUDCK2 (*kan⁺ lacIQ dck*:D133E)<br>pAK1 (*bla⁺ lacIQ adk⁺*) | Double transformation de CC103 |
| β7360 | *ara* Δ(*lac proB*)13<br>F' *lacZ*:Glu461Ala proB⁺ | Double transformation de CC104 |

(suite)

| Souche | Phénotype | Construction |
|---|---|---|
| | pSUDCK2 (*kan+ lacIQ dck:* D133E) | |
| | pAK1 (*bla+ lacIQ adk+*) | |
| β7361 | *ara Δ(lac proB)*13 | Double transformation de CC105 |
| | F' *lacZ:Glu*461Val *proB+* | |
| | pSUDCK2 (*kan+ lacIQ dck: D 133E*) | |
| | pAK1 (*bla+ lacIQ adk+*) | |
| β7362 | *ara Δ(lac proB)13* | Double transformation de CC106 |
| | F' lac*Z:*Glu461Lys *proB+* | |
| | pSUDCK2 (*kan+ lacIQ dck:*D133E) | |
| | pAK1 (*bla+ lacIQ adk+*) | |
| **Plasmides** | | |
| pTrc99A | *bla+ lacIQ* | ColE1 réplicon (Pharmacia) |
| pDCK1 | *bla+ lacIQ dck+* | Bouzon & Marlière |
| pDCK2 | *bla+ lacIQ dck:*D133E | Mutagénèse *In vivo* / disoG de β7134 |
| pDCK3 | *bla+ lacIQ dck:*R104Q | Mutagénèse *In vivo* / disoA de β7134 |
| pDCK4 | *bla+ lacIQ dck:*D133E-R104Q | Substitution d'un fragment *Sac*I-*BamH*I de 466 bases de pDCK3 par celui de pDCK2 |
| pSUTrc | *kan+ lacIQ* | Clonage du fragment *Sph*I-*BamH*I de pTrc99A dans pSU39 |
| pSUDCK1 | *kan+ lacIQ dck+* | Clonage du fragment *Sph*I-*BamH*I de pDCK1 dans pSU39 |
| pSUDCK2 | *kan+ lacIQ dck:* D133E | Clonage du fragment *Sph*I-*BamH*I de pDCK2 dans pSU39 |
| pSUDCK3 | *kan+ lacIQ dck:* R104Q | Clonage du fragment *Sph*I-*BamH*I de pDCK3 dans pSU39 |
| pSUDCK4 | *kan+ lacIQ dck:*D133E-R104Q | Clonage du fragment *Sph*I-*BamH*I de pDCK4 dans pSU39 |
| pAK1 | *bla+ lacIQ adk+* | Dr T. Okajima |
| pAKT39A | *bla+ lacIQ adk:*T39A | Dr. T. Okajima |

La souche β7069 a été sélectionnée comme étant un mutant spontané résistant à l'AZT de MG1655 cultivée sur boîte LB supplémentée avec 30 μM d'AZT. Le phénotype *tdk* a été déterminé par l'absence de croissance sur milieu riche Mueller-Hinton (MH) comprenant du triméthoprime et de la thymidine. On a contrôlé que la mutation β7069 tdk ne réverse pas spontanément en repiquant les cellules sur le milieu MH supplémenté avec du triméthoprime et de la thymidine après 20 générations en LB. La souche β7117 Δ*deo tdk* a été obtenue après deux transductions P1 consécutives. Les bactéries β7069 ont d'abord été infectées avec le lysat P1 provenant de KU8 dans le but de transférer la délétion Δ*serB* et le marqueur proximal *Tn10*. Les clones résistants à la tétracycline ont été sélectionnés et testés pour leur auxotrophie à la sérine. Un de ces clones a ensuite été infecté avec le lysat P1 provenant de SØ928 et les prototrophes à la sérine ont été sélectionnés sur milieu minimum. Tous les transductants Ser+ sélectionnés comportent la délétion de l'opéron *deo* car ils sont incapables de croître en milieu minimum avec de la thymidine comme seule source de carbone. Le plasmide pDCK4 a été construit en substituant le fragment SacI-BamHI de 466 bases de long de pDCK3 par celui de pDCK2. La présence des mutations D133E et R104Q sur pDCK4 a été déterminée par séquençage.

**Sélection *in vivo* des mutants DCK** : une culture de 12 heures des cellules β7134 en milieu MS supplémenté avec 50 mg/l de carbenicilline a été diluée 100 fois dans le même milieu et cultivée en aération jusqu'au moment où une turbidité de 0,100 (600nm) soit atteinte. La culture a ensuite été diluée 25 fois dans le même milieu avec ou sans IPTG et supplémentée avec des concentrations variables (5 à 30μM) des analogues de nucléosides promutanégiques disoG et disoA puis cultivée en aération pendant 18 heures. Les cellules ont été rincées puis étalées sur des boîtes MH supplémentées avec du triméthoprime, de la thymidine et de l'IPTG. Des colonies sont apparues après 36 heures d'incubation à 37°C. Aucune colonie n'a été obtenue en l'absence d'analogue de nucléoside.

**Test de détermination de la concentration minimale inhibitrice (CMI)** : la CMI des analogues de nucléosides a été déterminée conformément aux méthodes classiques avec les modifications suivantes : une culture bactérienne de 12 heures en milieu MS avec les antibiotiques appropriés a été diluée 100 fois dans le même milieu et cultivée à 37°C en aération jusqu'à l'obtention d'une turbidité d'environ 0,1 OD. (600nm). La culture a ensuite été diluée 100 fois dans le même milieu supplémenté avec IPTG et distribuée sur microplaque 96 puits dans un volume final de 100 μl dans une série de dilutions de 2 en 2 d'analogues de nucléotide. Chaque analogue a été testé deux fois. Après une incubation pendant 18 heures à 37°C avec agitation, le CMI a été déterminé comme correspondant à la concentration la plus faible en analogue pour laquelle aucune turbidité n'est détectable.

**Test de réversion :** Des cellules des souches lac CC101 à CC106 ont été transformées, soit avec le plasmide pSUDCK2 seul, soit avec les plasmides pSUDCK2 et pAK1. Les transformations ont été cultivées pendant 12 heures en milieu minimum comprenant 0,2% de glucose avec les antibiotiques appropriés pour la maintenance des plasmides. La culture a ensuite été diluée 100 fois dans le même milieu et cultivée jusqu'à l'apparition d'une DO de 0,1 (600nm). Ensuite, les cultures ont été diluées 100 fois dans le même milieu supplémenté avec 0,5 mM IPTG puis diluées 10 fois avec une solution 10 fois concentrée de l'analogue de nucléoside à tester. Les cellules ont été cultivées pendant 18 heures à 37°C avant la mise en culture sur boîte. Le dénombrement des cellules viables a été réalisé sur milieu solide LB. Les révertants Lac+ ont été sélectionnés en milieu minimum contenant 0,2% de lactose comme source de carbone. La fréquence de mutation a été définie comme étant le ratio : nombre de cellules mutantes sur nombre de cellules viables. Diverses concentrations finales des analogues nucléosidiques correspondant aux CMI/2, CMI/4, CMI/10 et CMI/20 ont été testées, car la sensibilité des souches dérivées de celles de Miller vis-à-vis des différents analogues n'est pas identique à celle des souches dérivées de MG1655. La fréquence de mutations a été déterminée en utilisant les cultures obtenues avec la concentration la plus élevée en analogue nucléosidique permettant une croissance visible après 18 heures.

## Exemple 1 : crible sélectif

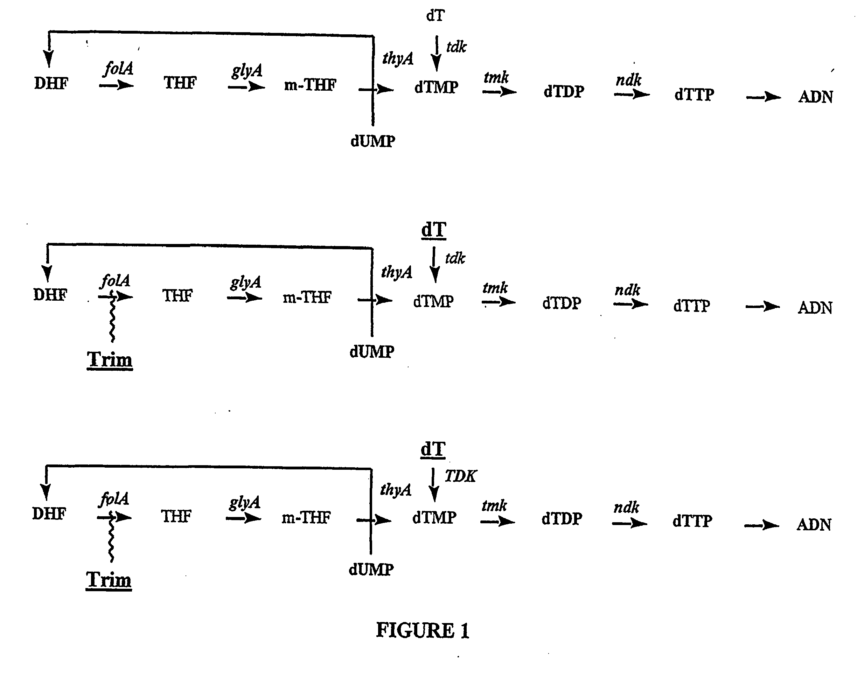

*Escherichia coli* ne possède pas d'activité désoxynucléoside kinase à l'exception d'une thymidine kinase, codée par le gène tdk, très spécifique de la thymidine et de la désoxyuridine. Nous avions montré précédemment comment l'introduction de l'activité désoxycytidine kinase chez *E. coli* ouvre une voie métabolique inexistante dans cet organisme, et permet aux désoxynucléosides naturels et à des analogues structuraux d'accéder au pool des monomères de l'ADN [Bouzon & Marlière, 1997]. Une souche portant un allèle défectif du gène tdk ne peut utiliser la thymidine comme source de thymidylate (dTMP) et sa croissance dépend de l'intégrité de la voie de synthèse *de novo* de celui-ci (Figure 1). La synthèse de dTMP à partir de dUMP est catalysée par la thymidylate synthase (gène thyA). Elle peut être bloquée par le triméthoprime qui par inhibition de l'activité de la dihydrofolate réductase conduit à l'épuisement du pool intracellulaire du 5,10-méthylène-tétrahydrofolate, donneur du groupement méthyle dans la réaction. Nous avons d'abord montré qu'une souche dont le gène tdk est inactivé ne prolifère pas sur milieu riche en présence de triméthoprime et de thymidine. Ce crible sélectif qui impose le maintien d'une activité thymidine kinase a été mis en oeuvre pour faire évoluer l'activité de la désoxycytidine kinase humaine chez *E. coli*. On le trouve schématisé dans la Figure 1.

## Exemple 2 : sélection de mutants dckH présentant une activité élargie

Nous avons mis à profit la propriété de mutateur conditionnel de DCK en présence d'analogues de nucléosides promutagènes pour soumettre son propre gène dck à un épisode de mutagénèse *in vivo*. Ainsi, des bactéries de génotype Δ*deo tdk p:: dckH*+ furent exposées soit à la 2'-désoxy-iso-adénosine (disoA) soit à la 2'-désoxy-iso-guanosine (disoG), puis incubées sur milieu solide riche en présence de triméthoprime et de thymidine. Des colonies sont apparues suite à l'administration des deux composés à une fréquence de l'ordre de $10^{-8}$. Aucune colonie ne survit en absence de nucléoside promutagène.

Le séquençage des gènes de 7 plasmides mutants obtenus indépendamment (4 après mutagénèse par disoG, 3 par disoA) mit en évidence deux mutations ponctuelles D133E et R104Q, chacune conférant l'acquisition de l'activité thymidine kinase par DCK. Ces allèles sont désignés dckH*. Un plasmide réunissant les deux mutations dans un même allèle, dckH**, fut construit et introduit dans la souche β7117 de génotype Δ*deo tdk*. Il permit la complémentation de la mutation tdk, exprimant donc aussi une activité thymidine kinase. Les différents allèles sélectionnés sont répertoriés dans le Tableau 2 ci-après.

**Tableau 2: mutations du gène hétérologue dckH supprimant le phénotype tdk de *Escherichia coli*.**

| Mutagène | Mutation détectée | Fréquence | Site de la mutation | substitution acide aminé |
|---|---|---|---|---|
| disoG | C → A | 4/4 | codon 133 GAC | Asp -> Glu |
| disoA | C → A | 1/3 | codon 133 GAC | Asp -> Glu |

(suite)

| Mutagène | Mutation détectée | Fréquence | Site de la mutation | substitution acide aminé |
|---|---|---|---|---|
| | C → G | 1/3 | codon 133 GAC | Asp -> Glu |
| | G → A | 1/3 | codon 104 CGA | Arg -> Gln |

Les détails de la sélection et de l'analyse génétique sont donnés dans la section Matériels et Méthodes.

**Exemple 3 : propriétés fonctionnelles des mutants dckH**

La toxicité d'analogues de nucléosides, déviant soit par le sucre soit par la base, a été évaluée dans des souches de génotype Δdeo tdk cdd exprimant sur un plasmide les différents allèles de dckH, allèle sauvage, D133E, R104Q et double mutant.

Le marqueur Δdeo correspond à l'inactivation de l'opéron catabolique des désoxynucléosides et le marqueur cdd à l'inactivation de la désoxycytidine désaminase; ces marqueurs évitent que les analogues de nucléosides apportés dans le milieu engendrent des dérivés autres que le dérivé phosphorylé souhaité par action de DCK; ils permettent l'emploi de doses plus faibles des analogues. Les résultats sont indiqués dans le Tableau 3 ci-après.

**Tableau 3: toxicité d'analogues de deoxynucléoside induite par la deoxycytidine kinase sur *Escherichia coli*.**

| Analogue de nucléoside | Souche et allèle dck | | | | |
|---|---|---|---|---|---|
| | Δdeo tdk cdd:: Tn 10 | | | | |
| | β7334 | β7335 | β7336 | β7337 | β7338 |
| | none | wt | D133E | R104Q | D133E R104Q |
| ddA | 80 | 80 | 80 | 80 | 80 |
| ddU | > | > | > | > | > |
| ddT | > | > | > | > | > |
| ddC | > | > | > | > | > |
| ddI | 80 | 40 | 40 | 20 | 40 |
| araC | > | 160 | 20 | 20 | 320 |
| AZT | > | > | 640 | 640 | 1280 |
| 5'-amino-dT | > | > | > | > | > |
| disoA | > | > | 10 | > | > |
| disoG | > | > | > | > | > |
| dI | > | > | 1.25 | > | 320 |
| disoI | > | > | > | > | > |
| 8ho'dI (*) | > | > | 1280 | > | > |
| DAP | > | > | > | > | > |
| d-oxanosine | > | > | 80 | > | > |
| dY | > | > | > | > | > |
| dJ | > | > | > | > | > |
| amino-dC | > | > | 20 | 2.5 | 20 |
| 5-aza-dC | > | 1.25 | 1.25 | 1.25 | 1.25 |
| 5-iodo-dC | > | > | > | > | > |
| 5-bromo-dC | > | > | > | > | > |
| 5-methyl-dC | > | > | > | > | > |
| 5-methyl-disoC | > | > | > | > | > |
| 5-chloro-dU | > | > | > | > | > |
| 5-bromo-dU | > | > | > | > | > |
| 5-iodo-dU | > | > | > | > | > |

(suite)

| Analogue de nucléoside | Souche et allèle dck | | | | |
|---|---|---|---|---|---|
| | | | Δ*deo tdk cdd:: Tn* 10 | | |
| | β7334 | β7335 | β7336 | β7337 | β7338 |
| | none | wt | D133E | R104Q | D133E R104Q |
| 5-hm-dU | > | > | > | > | > |

(*) désoxyribonucléoside de la 8-hydroxy-hypoxanthine

Les concentrations inhibitrices minimales, exprimées en microM, ont été déterminées comme indiqué dans la section Matériels et Méthodes.

Chaque dosage a été effectué trois fois : aucune toxicité n'a pu être détectée à plus haute concentration en analogue, 1,28 mM.

Le génotype détaillé des souches hôtes de chaque allèle est indiqué au Tableau 1.

Bien que conduisant toutes deux à l'acceptation de la thymidine comme substrat, les mutations ponctuelles D133E et R104Q ont des effets contrastés sur la phosphorylation des différents analogues testés.

Les souches sauvages d'*E. coli* sont sensibles à de faibles concentrations d'AZT, tandis que les souches tdk, qui ont perdu l'activité thymidine kinase, y sont réfractaires [Elwell et al, 1987]. Les tests rapportés dans le Tableau 3 indiquent que l'AZT n'est pas substrat de DCK wt, mais que le mutant D133E active l'analogue de telle façon qu'on détecte une toxicité à haute concentration de l'analogue (CMI= 1280 microM). Il est probable que cette toxicité provient de l'incorporation de l'AZT triphosphate par l'ADN polymérase et le blocage de l'élongation par ce terminateur de chaine après les actions successives de la dTMP kinase et de la nucléoside diphosphokinase sur l'AZT monophosphate.

Poursuivant l'analyse des résultats du Tableau 3, la mutation D133E entraîne une forte toxicité de disoA, (CMI= 10 microM). La mutation R104Q n'a pas d'effet vis-à-vis de ce composé. De même, la mutation D133E entraîne une très forte toxicité de la désoxyinosine, dI (CMI= 1 microM). Le désoxyribonucléoside de la 2-hydroxy-4-hydrazino-pyrimidine (désignée 4am'dC) apparait meilleur substrat du mutant R104Q que du mutant D133E, les deux mutations causant une très forte augmentation de la toxicité de l'analogue. La 5-aza-désoxycytidine (désignée 5azadC) est toxique à très faible concentration (CMI< 1.25 microM) quel que soit l'allèle de DCK.

Globalement, l'allèle dckH-D133E se montre le plus intéressant, augmentant la sensibilité pour le plus grand nombre d'analogues. La combinaison des deux mutations D133E et R104Q mène à un spectre d'activité apparemment intermédiaire entre chacun des deux mutants individuels.

**Exemple 4 : diversification métabolique par coexpression de gènes hétérologues**

L'absence d'effet toxique par un analogue de nucléoside vis-à-vis d'une souche d'*E. coli* exprimant le gène dckH ou l'un de ses allèles mutants peut avoir plusieurs raisons, si on suppose que tout effet toxique résulte de l'incorporation de monomères erronés dans les chaînes d'ADN : (i) l'analogue n'est pas substrat ou est un mauvais substrat de l'enzyme DCK; (ii) l'analogue est phosphorylé en monophosphate par DCK mais les étapes ultérieures de phosphorylation en diphosphate puis en triphosphate échouent; (iii) l'analogue triphosphate n'est pas substrat de l'ADN polymérase.

Il est connu que les nucléoside monophosphate kinases, qui produisent les diphosphates à partir des triphosphates acceptent le ribose et le désoxyribose, mais sont très spécifiques de la base. On s'attendait donc à ce que la coproduction d'un enzyme formant une variété élargie de monophosphates (allèles mutés DCK) et d'un enzyme formant une variété élargie de diphosphates dans la même cellule d'*E. coli* révèle les substrats nucléosidiques portant des bases déviantes susceptibles d'être phosphorylés en monophosphates par DCK wt ou DCK muté mais dont la conversion en diphosphate ne puisse être catalysée par les enzymes d'*E. coli.*

L'adénosine monophosphate kinase des eucaryotes (AMK) présente une parenté de structure avec les UMP/CMP kinases des bactéries [Okajima et al, 1993]. Sa fonction physiologique serait de catalyser l'échange de phosphate entre AMP et ATP:

$$AMP + ATP <--> ADP + ADP ;$$

l'enzyme agit également sur un substrat portant le désoxyribose:

$$dAMP + ATP <--> dADP + ADP .$$

Le mutant T39A du gène de l'enzyme du poulet (amkG*) a été construit par mutagénèse dirigée en se guidant sur des comparaisons de séquence, par un groupe au Japon [Okajima et al, 1993]. In vitro, l'activité de AMK sur CMP est inférieure à 1 % de son activité sur AMP. La mutation T39A modifie le spectre d'activité de l'enzyme et augmente significativement la conversion de CMP et UMP [Okajima et al, 1993].

Nous avons exprimé conjointement dans le contexte génétique Δ*deo tdk cdd* chacun des quatre allèles de dckH avec chacun des deux allèles wt et T39A de amkG, et testé la toxicité des différents analogues de nucléosides déjà testés précédemment (Tableau 4 ci-après).

**Tableau 4: toxicité d'analogues de deoxynucléoside induite par la coexpression de la deoxycytidine kinase and de l'adenosine monophosphate kinase dans *Escherichia coli.***

| Analogue de Nucléoside | Strain dckH allele amkG allele | | | | | | | | | |
|---|---|---|---|---|---|---|---|---|---|---|
| | β7339 none wt | β7340 wt wt | β7341 D133 E wt | β7342 R104 Q wt | β7343 D133E R104Q wt | β7344 none T39A | β7345 wt T39A | β7346 D133 E T39A | β7347 R104 Q T39A | β7348 D133E R104Q T39A |
| AZT | > | > | > | > | > | > | > | 1280 | > | > |
| disoA | > | > | 5 | > | 1280 | > | > | 10 | > | > |
| disoG | > | > | > | > | > | > | > | > | > | > |
| dI | > | > | ≤1.25 | > | 1280 | > | > | ≤1.25 | > | 1280 |
| 8oxodI | > | > | 1280 | > | > | > | > | 1280 | > | > |
| dY | > | > | > | > | > | > | > | 1280 | > | > |
| 4am'dC | > | > | 80 | ≤1.25 | 80 | > | > | 640 | 5 | 320 |
| 5azadC | > | ≤1.25 | ≤1.25 | ≤1.25 | ≤1.25 | > | ≤1.25 | ≤1.25 | ≤1.25 | ≤1.25 |
| 5IdC | > | > | ≤1.25 | ≤1.25 | ≤1.25 | > | ≤1.25 | ≤1.25 | ≤1.25 | ≤1.25 |
| 5BrdC | > | ≤1.25 | ≤1.25 | ≤1.25 | ≤1.25 | > | ≤1.25 | ≤1.25 | ≤1.25 | ≤1.25 |
| 5MedC | > | ≤1.25 | ≤1.25 | ≤1.25 | 5 | > | ≤1.25 | ≤1.25 | ≤1.25 | ≤1.25 |

Les concentrations inhibitrices minimum, exprimées en microM, ont été déterminées comme indiqué dans la section Matériels and méthodes.

Chaque expérience a été effectuée trois fois : aucune toxicité n'a pu être détectée à plus haute concentration en analogue, 1280 microM.

Le génotype détaillé des souches hôtes de chaque allèle est indiqué au Tableau 3.

Il est apparu que la coexpression des deux gènes eucaryotes entraîne la conversion métabolique des dérivés 5-halogénés (5BrdC, 5IdC) et 5-méthylé (5MedC) de la désoxycytidine dC en dérivés inhibiteurs pour les bactéries recombinantes, tandis que l'expression d'un seul des deux gènes laisse les bactéries réfractaires aux mêmes analogues. La très grande toxicité de l'analogue lorsqu'il y a expression concomitante de DCK et AMK indique que DCK phosphoryle ces substrats mais que c'est l'étape ultérieure de phosphorylation par les monophosphates kinases qui est limitante.

Comme on le voit dans le Tableau 4, la conjonction de l'allèle DCK-D133E et de l'allèle AMK-T39A entraîne une toxicité des souches d'*E. coli* qui les portent vis-à-vis du nucléoside simplifié dY, désoxyribosyl-imidazole-carboxamide [Pochet et al, 1995]. Les effets mutagènes de la base Y avaient été démontrés ex vivo au cours de réactions d'amplification PCR, causant en particulier des transitions A:T->G:C et des transversions A:T->T:A [Sala et al, 1996]. La toxicité de dY à 1 mM vis-à-vis des souches rapportées ici s'accompagne d'une augmentation du même spectre de mutations *in vivo*.

**REFERENCES**

Bouzon M. & Marliere P. (1997) Human deoxycytidine kinase as a conditional mutator in Escherichia coli. Comptes Rendus de 1 Academie des Sciences - Serie III, Sciences de la Vie. 320(6):427-34

Brown DG. Visse R. Sandhu G. Davies A. Rizkallah PJ. Melitz C. Summers WC. Sanderson MR. (1995) Crystal structures of the thymidine kinase from herpes simplex virus type-1 in complex with deoxythymidine and ganciclovir. Nature Structural Biology. 2(10):876-81

Cazaux C, Tiraby M, Loubiere, Haren L, Klatzmann D & Tiraby G (1998) Phosphorylation and cytotoxicity of therapeutic nucleoside analogues: a comparison of alpha and gamma herpesvirus thymidine kinase suicide genes. Cancer Gene Therapy 5(2):83-91

Chottiner EG. Shewach DS. Datta NS. Ashcraft E. Gribbin D. Ginsburg D. Fox IH. Mitchell BS. (1991) Cloning and expression of human deocytidine kinase cDNA Proceedings of the National Academy of Sciences of the United States of America. 88(4):1531-5

Datta NS. Shewach DS. Hurley MC. Mitchell BS. Fox IH. (1989) Human T-lymphoblast deoxycytidine kinase: purification and properties. Biochemistry. 28(1):114-23

Elwell LP et al (1987) Antibacterial activity and mechanism of action of 3'-azido-3'-deoxythymidine (BW A509U). Antimicrobial Agents & Chemotherapy 31(2):274-80

Harrison PT. Thompson R. Davison AJ. (1991) Evolution of herpesvirus thymidine kinases from cellular deoxycytidine kinase. Journal of General Virology. 72:2583-6

Johansson M, Van Rompay AR, Degrève B, Balzarini J & Karlsson A (1999) Cloning and characterization of the multisubstrate deoxyribonucleoside kinase of Drosophila melanogaster. J Biol Chem 274:23814-23819

Mullen CA (1994) Metabolic suicide genes in gene therapy. [Review] Pharmacology & Therapeutics 63(2):199-207

Okajima T. Tanizawa K. Fukui T. (1993) Site-directed mutagenesis of AMP-binding residues in adenylate kinase. FEBS Letters. 334(1):86-8

Pochet S., Dugué L., Meier A. & Marlière P. (1995) "Enzymatic synthesis of 1-(2-deoxy-β-D-ribofuranosyl)-imidazole-4-carboxamide, a simplified DNA building block" Bioorganic & Medicinal Chemistry Letters 5, 1679-1684.

Sala M., Pezo V., Pochet S. & Wain-Hobson S. (1996) "Ambiguous base pairing of the purine analogue 1-(2-deoxy-β-D-ribofuranosyl)-imidazole-4-carboxamide during PCR" Nucleic Acids Research 24, 3302-3306.

**Revendications**

1.  Procédé pour faire évoluer une protéine X de sorte à en modifier ses caractéristiques comprenant les étapes suivantes :

    a) obtention de cellules comportant un génotype [protéine Y∗ : :protéine X+], par transformation de cellules [protéine Y∗] avec un acide nucléique comprenant le gène codant pour la protéine X, Y∗ signifiant que le gène codant pour Y a été inactivé, Y étant une protéine appartenant à une classe voisine de X possédant une activité voisine, les classes de X et Y **se caractérisant en ce qu'**elles possèdent au moins les trois premiers chiffres appartenant aux classes EC de la nomenclature internationale à 4 chiffres, lesdites cellules présentant un phénotype auxotrophe nécessitant pour survivre l'apport du produit de la réaction de Y sur son substrat dans le milieu de culture ;
    b) exposer les cellules obtenues à l'étape a) à un mutagène,
    c) mettre en culture lesdites cellules dans un milieu comprenant le substrat de Y, le produit de la réaction de Y sur son substrat étant nécessaire à la survie de ladite cellule,
    d) sélectionner les cellules qui ont survécu à l'étape c) dans lesquelles la protéine X, modifiée par l'action dudit analogue nucléoside promutagène, complémente la déficience en la protéine Y.

2.  Procédé selon la revendication 1 **caractérisé en ce que** la protéine X est sélectionnée parmi les kinases appartenant aux classes EC 2.7.1.- , les nucléotidyl transférases appartenant aux classes EC 2.7.7.-, notamment les polymérases et les phosphorylases appartenant aux classes EC 2.4.2.-..

3.  Procédé selon la revendication 2 **caractérisé en que** en ce que la protéine X est capable d'activer un analogue de nucléosides promutagène, lequel analogue introduit des mutations dans son propre gène.

4.  Procédé selon l'une des revendications 1 à 3 pour faire évoluer une kinase X de sorte à en modifier ses caractéristiques **caractérisé en ce qu'**il comprend les étapes suivantes :

    a) obtention de cellules comportant un génotype [kinase Y∗ : :kinaseX+], par transformation d'une cellule [kinase Y*] avec un acide nucléique comprenant le gène codant pour la kinase X, Y∗ signifiant que le gène codant pour Y a été inactivé, Y étant une kinase appartenant à une classe voisine de X montrant une activité voisine, les classes de X et Y **se caractérisant en ce qu'**elles possèdent au moins les trois premiers chiffres appartenant aux classes EC 2.7.1.- de la nomenclature internationale à 4 chiffres, lesdites cellules présentant un phénotype auxotrophe nécessitant pour survivre l'apport du produit de la réaction de Y sur son substrat dans le milieu de culture ;

b) exposer les cellules obtenues à l'étape a) à un analogue de nucléosides promutagènes pendant un temps donné, la kinase X étant capable de phosphoryler ledit analogue,

c) mettre en culture lesdites cellules dans un milieu comprenant le substrat de Y, le produit de la réaction de Y sur son substrat étant nécessaire à la survie de ladite cellule,

d) sélectionner les cellules qui ont survécu à l'étape c) dans lesquelles la kinase X, modifiée par l'action dudit agent mutagène, complémente la déficience en la kinase Y.

5. Procédé selon l'une des revendications 1 à 4 **caractérisé en ce que** lesdites cellules sont des cellules procaryotes ou eucaryotes, de préférence *E. coli.*

6. Procédé selon l'une des revendications 1 à 5 **caractérisé en ce que** ledit substrat est sélectionné parmi les nucléosides et leurs analogues.

7. Procédé selon l'une des revendications 4 à 6 **caractérisé en ce que** la kinase X est une déoxycytidine kinase (DCK) appartenant à la classe EC 2.7.1.74.

8. Procédé selon l'une des revendications 4 à 7 **caractérisé en ce que** la kinase Y est une thymidine kinase (TDK) appartenant à la classe EC 2.7.1.21.

9. Procédé selon l'une des revendications 1 à 5 **caractérisé en ce qu'**il comprend les étapes suivantes :

a) obtention d'une bactérie *E. coli Δdeo tdk p::dckH+,* mise en culture des cellules obtenues à l'étape a) dans un milieu comprenant :

- un agent mutagène sélectionné parmi les analogues de nucléosides promutagènes et du triméthoprime qui bloque la synthèse du thymidylate par la thymidylate synthase ;
- et de la thymidine qui est nécessaire à la survie de ladite cellule,

b) sélection des cellules qui ont survécu à l'étape b) dans lesquelles la DCKH, modifiée par l'action dudit analogue promutagène, complémente la déficience en TDK.

10. Procédé selon l'une des revendications 4 à 9 **caractérisé en ce que** la kinase X est une désoxycytidine kinase, de préférence la DCK1 humaine possédant la séquence déposée dans GENBANK sous le numéro d'accession M60527 comportant au moins une mutation sélectionnée parmi les mutations D133E et R104Q.

11. Protéine X mutée susceptible d'être obtenue à partir du procédé selon l'une des revendications 1 à 10 **caractérisée en ce qu'**elle présente une activité élargie par rapport à la Protéine X initiale.

12. Kinase X mutée susceptible d'être obtenue à partir du procédé selon l'une des revendications 1 à 10 **caractérisée en ce qu'**elle présente une activité élargie par rapport à la kinase X initiale.

13. Kinase selon la revendication 12 **caractérisée en ce qu'**il s'agit de la DCK1 humaine possédant la séquence déposée dans GENBANK sous le numéro d'accession M60527 comportant au moins une mutation sélectionnée parmi les mutations D133E et R104Q.

14. Acide nucléique comprenant la séquence codante pour la kinase selon la revendication 13.

15. Vecteur d'expression comprenant la séquence codante pour la kinase selon la revendication 13.

16. Vecteur selon la revendication 15 **caractérisé en ce que** ladite séquence est fusionnée à un promoteur efficace dans les cellules eucaryotes et/ou procaryotes.

17. Vecteur selon l'une des revendications 15 et 16 **caractérisé en ce qu'**il s'agit d'un plasmide capable de transformer et de se maintenir chez *E. coli.*

18. Cellule hôte comprenant un vecteur selon l'une des revendications 15 à 17.

19. Utilisation d'une kinase selon l'une des revendications 12 et 13 dans un procédé selon l'une des revendications 1 à 10 pour l'activation dudit analogue promutagène.

20. Utilisation d'une kinase selon l'une des revendications 12 et 13 dans un procédé d'hypermutagénèse.

21. Utilisation d'une kinase selon l'une des revendications 12 et 13 pour convertir des nucléosides, naturellement réfractaires à la phosphorylation enzymatique, en leur dérivé 5' phosphate respectif.

22. Utilisation concomitante d'une kinase selon l'une des revendications 12 et 13 et d'autres enzymes tels que AMK et/ou la transdesoxyribosylase (NTD) pour élargir in *vivo* la gamme de la mutagénèse à l'aide de promutagènes.

23. Utilisation d'un vecteur selon l'une des revendications 15 à 17 pour la préparation d'un médicament destiné à la thérapie génique pour permettre l'incorporation d'analogues de nucléosides dans l'ADN.

24. Procédé de mutagénèse *in vivo* d'une séquence d'ADN déterminée, ladite séquence d'ADN étant dans une cellule, comprenant les étapes consistant à :

- effectuer la mutation par insertion d'au moins un type de nucléosides promutagènes dans ladite séquence, la cellule exprimant au moins un système enzymatique permettant l'insertion dudit nucléotide promutagène dans l'ADN,
- et détecter la présence de la séquence mutée,

**caractérisé en ce que** le système enzymatique comprend une kinase selon l'une des revendications 12 et 13.

25. Procédé selon la revendication 22 pour faire évoluer une protéine spécifique, ladite protéine étant exprimé de la cellule, **caractérisé en ce qu'**on inactive un gène codant pour une protéine apparentée à ladite protéine spécifique, la protéine apparentée étant nécessaire à la survie de la cellule, et on détecte la complémentation après mutation du gène codant pour ladite protéine spécifique.

26. Procédé selon la revendication 25 **caractérisé en que** ladite protéine spécifique et ladite protéine apparentée sont des enzymes appartenant à une classe voisine ayant en commun au moins les trois premiers chiffres de la nomenclature internationale EC à 4 chiffres.

27. Procédé selon la revendication 26 **caractérisé en que** lesdites protéines ont la propriété de pouvoir faire évoluer leur propre gène.

28. Souche d'*E. coli* β 7151 de génotype Δ*deo tdk* comprenant un vecteur exprimant la DCK mutée D133E déposée à la CNCM sous le numéro d'accession I-2631.

29. Souche d'*E. coli* β 7134 de génotype Δ*deo tdk* comprenant un vecteur exprimant la DCK humaine déposée à la CNCM sous le numéro d'accession I-2630.

30. Souche d'*E. coli* β7338 de génotype Δ*deo tdk* comprenant un vecteur exprimant la DCK double mutante D133E et R104Q déposée à la CNCM sous le numéro d'accession I-2650.

FIGURE 1

EP 1 921 143 A2

dA $\longrightarrow$ dAMP

dC $\longrightarrow$ dCMP

*dck*

dG $\longrightarrow$ dGMP

dT ✕$\rightarrow$ dTMP

dA ✕$\rightarrow$ dAMP

dC ✕$\rightarrow$ dCMP

*tdk*

dG ✕$\rightarrow$ dGMP

dT $\longrightarrow$ dTMP

FIGURE 2

AZT

disoA

disoG

dI

8ho'dI

dY

4am'dC

5azadC

5IdC

5BrdC

5MedC

**FIGURE 3**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 6015705 A
- US 6063376 A

**Littérature non-brevet citée dans la description**

- **HALL et al.** *Protein Eng.,* 1991, vol. 4, 601
- **HEMSLEY et al.** *Nucleic Acids Research,* 1989, vol. 17, 6545-6551
- **HO et al.** *Gene,* 1989, vol. 77, 51-59
- **HULTMAN et al.** *Nucleic Acids Research,* 1990, vol. 18, 5107-5112
- **JONES et al.** *Nature,* 1990, vol. 344, 793-794
- **JONES et al.** *Biotechniques,* 1992, vol. 12, 528-533
- **LANDT et al.** *Gene,* 1990, vol. 96, 125-128
- **NASSAL et al.** *Nucleic Acids Research,* 1990, vol. 18, 3077-3078
- **NELSON et al.** *Analytical Biochemistry,* 1989, vol. 180, 147-151
- **VALLETTE et al.** *Nucleic Acids Research,* 1989, vol. 17, 723-733
- **WATKINS et al.** *Biotechniques,* 1993, vol. 15, 700-704
- **WEINER et al.** *Gene,* 1993, vol. 126, 35-41
- **YAO et al.** *PCR Methods and Applications,* 1992, vol. 1, 205-207
- **WEINER et al.** *Gene,* 1994, vol. 151 (1/9), 123
- **BOUZON M. ; MARLIERE P.** Human deoxycytidine kinase as a conditional mutator in Escherichia coli. Comptes Rendus de 1 Academie des Sciences - Serie III. *Sciences de la Vie,* 1997, vol. 320 (6), 427-34
- **BROWN DG. ; VISSE R. ; SANDHU G. ; DAVIES A. ; RIZKALLAH PJ. ; MELITZ C. ; SUMMERS WC. ; SANDERSON MR.** Crystal structures of the thymidine kinase from herpes simplex virus type-1 in complex with deoxythymidine and ganciclovir. *Nature Structural Biology.,* 1995, vol. 2 (10), 876-81
- **CAZAUX C ; TIRABY M ; LOUBIERE ; HAREN L ; KLATZMANN D ; TIRABY G.** Phosphorylation and cytotoxicity of therapeutic nucleoside analogues: a comparison of alpha and gamma herpesvirus thymidine kinase suicide genes. *Cancer Gene Therapy,* 1998, vol. 5 (2), 83-91
- **CHOTTINER EG. ; SHEWACH DS. ; DATTA NS. ; ASHCRAFT E. ; GRIBBIN D. ; GINSBURG D. ; FOX IH. ; MITCHELL BS.** Cloning and expression of human deocytidine kinase cDNA. *Proceedings of the National Academy of Sciences of the United States of America.,* 1991, vol. 88 (4), 1531-5
- **DATTA NS. ; SHEWACH DS. ; HURLEY MC. ; MITCHELL BS. ; FOX IH.** Human T-lymphoblast deoxycytidine kinase: purification and properties. *Biochemistry,* 1989, vol. 28 (1), 114-23
- **ELWELL LP et al.** Antibacterial activity and mechanism of action of 3'-azido-3'-deoxythymidine (BW A509U). *Antimicrobial Agents & Chemotherapy,* 1987, vol. 31 (2), 274-80
- **HARRISON PT. ; THOMPSON R. ; DAVISON AJ.** Evolution of herpesvirus thymidine kinases from cellular deoxycytidine kinase. *Journal of General Virology.,* 1991, vol. 72, 2583-6
- **JOHANSSON M ; VAN ROMPAY AR ; DEGRÈVE B ; BALZARINI J ; KARLSSON A.** Cloning and characterization of the multisubstrate deoxyribonucleoside kinase of Drosophila melanogaster. *J Biol Chem,* 1999, vol. 274, 23814-23819
- **MULLEN CA.** Metabolic suicide genes in gene therapy. *Pharmacology & Therapeutics,* 1994, vol. 63 (2), 199-207
- **OKAJIMA T. ; TANIZAWA K. ; FUKUI T.** Site-directed mutagenesis of AMP-binding residues in adenylate kinase. *FEBS Letters.,* 1993, vol. 334 (1), 86-8
- **POCHET S. ; DUGUÉ L. ; MEIER A. ; MARLIÈRE P.** Enzymatic synthesis of 1-(2-deoxy-β-D-ribofuranosyl)-imidazole-4-carboxamide, a simplified DNA building block. *Bioorganic & Medicinal Chemistry Letters,* 1995, vol. 5, 1679-1684
- **SALA M. ; PEZO V. ; POCHET S. ; WAIN-HOBSON S.** Ambiguous base pairing of the purine analogue 1-(2-deoxy-β-D-ribofuranosyl)-imidazole-4-carboxamide during PCR. *Nucleic Acids Research,* 1996, vol. 24, 3302-3306

LISTE DE SEQUENCES

<110>  INSTITUT PASTEUR
       CENTRE NATIONAL DE LA RECHERCHE SCIENTIFIQUE   CNRS

<120>  Mutants de la désoxycytidine kinase possédant un activite
       enzymatique élargie

<130>  BIF 023265 PCT

<140>  PCT/FR02/01252
<141>  2002-04-10

<150>  FR 01 04856
<151>  2001-04-10

<160>  3

<170>  PatentIn version 3.2

<210>  1
<211>  260
<212>  PRT
<213>  Homo sapiens


<220>
<223>  DCK1 humaine D133E

<400>  1

Met Ala Thr Pro Pro Lys Arg Ser Cys Pro Ser Phe Ser Ala Ser Ser
1               5                   10                  15


Glu Gly Thr Arg Ile Lys Lys Ile Ser Ile Glu Gly Asn Ile Ala Ala
            20                  25                  30


Gly Lys Ser Thr Phe Val Asn Ile Leu Lys Gln Leu Cys Glu Asp Trp
        35                  40                  45


Glu Val Val Pro Glu Pro Val Ala Arg Trp Cys Asn Val Gln Ser Thr
    50                  55                  60


Gln Asp Glu Phe Glu Glu Leu Thr Met Ser Gln Lys Asn Gly Gly Asn
65                  70                  75                  80


Val Leu Gln Met Met Tyr Glu Lys Pro Glu Arg Trp Ser Phe Thr Phe
                85                  90                  95


Gln Thr Tyr Ala Cys Leu Ser Arg Ile Arg Ala Gln Leu Ala Ser Leu
            100                 105                 110

```
Asn Gly Lys Leu Lys Asp Ala Glu Lys Pro Val Leu Phe Phe Glu Arg
        115             120             125

Ser Val Tyr Ser Glu Arg Tyr Ile Phe Ala Ser Asn Leu Tyr Glu Ser
    130             135             140

Glu Cys Met Asn Glu Thr Glu Trp Thr Ile Tyr Gln Asp Trp His Asp
145             150             155             160

Trp Met Asn Asn Gln Phe Gly Gln Ser Leu Glu Leu Asp Gly Ile Ile
                165             170             175

Tyr Leu Gln Ala Thr Pro Glu Thr Cys Leu His Arg Ile Tyr Leu Arg
            180             185             190

Gly Arg Asn Glu Glu Gln Gly Ile Pro Leu Glu Tyr Leu Glu Lys Leu
        195             200             205

His Tyr Lys His Glu Ser Trp Leu Leu His Arg Thr Leu Lys Thr Asn
    210             215             220

Phe Asp Tyr Leu Gln Glu Val Pro Ile Leu Thr Leu Asp Val Asn Glu
225             230             235             240

Asp Phe Lys Asp Lys Tyr Glu Ser Leu Val Glu Lys Val Lys Glu Phe
            245             250             255

Leu Ser Thr Leu
            260


<210>   2
<211>   260
<212>   PRT
<213>   Homo sapiens


<220>
<223>   DCK1 humaine R104Q

<400>   2

Met Ala Thr Pro Pro Leu Ala Ser Cys Pro Ser Pro Ser Ala Ser Ser
1               5               10              15


Gly Gly Thr Ala Ile Leu Leu Ile Ser Ile Gly Gly Ala Ile Ala Ala
            20              25              30
```

```
Gly Leu Ser Thr Pro Val Ala Ile Leu Leu Gly Leu Cys Gly Ala Thr
        35              40              45

Gly Val Val Pro Gly Pro Val Ala Ala Thr Cys Ala Val Gly Ser Thr
    50              55              60

Gly Ala Gly Pro Gly Gly Leu Thr Met Ser Gly Leu Ala Gly Gly Ala
65              70              75              80

Val Leu Gly Met Met Thr Gly Leu Pro Gly Ala Thr Ser Pro Thr Pro
            85              90              95

Gly Thr Thr Ala Cys Leu Ser Gly Ile Ala Ala Gly Leu Ala Ser Leu
            100             105             110

Ala Gly Leu Leu Leu Ala Ala Gly Leu Pro Val Leu Pro Pro Gly Ala
        115             120             125

Ser Val Thr Ser Ala Ala Thr Ile Pro Ala Ser Ala Leu Thr Gly Ser
    130             135             140

Gly Cys Met Ala Gly Thr Gly Thr Thr Ile Thr Gly Ala Thr His Ala
145             150             155             160

Thr Met Ala Ala Gly Pro Gly Gly Ser Leu Gly Leu Ala Gly Ile Ile
            165             170             175

Thr Leu Gly Ala Thr Pro Gly Thr Cys Leu His Ala Ile Thr Leu Ala
            180             185             190

Gly Ala Ala Gly Gly Gly Gly Ile Pro Leu Gly Thr Leu Gly Leu Leu
    195             200             205

His Thr Leu His Gly Ser Thr Leu Leu His Ala Thr Leu Leu Thr Ala
    210             215             220

Pro Ala Thr Leu Gly Gly Val Pro Ile Leu Thr Leu Ala Val Ala Gly
225             230             235             240

Ala Pro Leu Ala Leu Thr Gly Ser Leu Val Gly Leu Val Leu Gly Pro
            245             250             255

Leu Ser Thr Leu
        260
```

```
<210>   3
<211>   260
<212>   PRT
<213>   Homo sapiens


<220>
<223>   DCK1 humaine D133E-R104Q

<400>   3

Met Ala Thr Pro Pro Lys Arg Ser Cys Pro Ser Phe Ser Ala Ser Ser
1               5                   10                  15


Glu Gly Thr Arg Ile Lys Lys Ile Ser Ile Glu Gly Asn Ile Ala Ala
            20                  25                  30


Gly Lys Ser Thr Phe Val Asn Ile Leu Lys Gln Leu Cys Glu Asp Trp
        35                  40                  45


Glu Val Val Pro Glu Pro Val Ala Arg Trp Cys Asn Val Gln Ser Thr
    50                  55                  60


Gln Asp Glu Phe Glu Glu Leu Thr Met Ser Gln Lys Asn Gly Gly Asn
65                  70                  75                  80


Val Leu Gln Met Met Tyr Glu Lys Pro Glu Arg Trp Ser Phe Thr Phe
                85                  90                  95


Gln Thr Tyr Ala Cys Leu Ser Gln Ile Arg Ala Gln Leu Ala Ser Leu
                10.0                 105                 110


Asn Gly Lys Leu Lys Asp Ala Glu Lys Pro Val Leu Phe Phe Glu Arg
            115                 120                 125


Ser Val Tyr Ser Glu Arg Tyr Ile Phe Ala Ser Asn Leu Tyr Glu Ser
    130                 135                 140


Glu Cys Met Asn Glu Thr Glu Trp Thr Ile Tyr Gln Asp Trp His Asp
145                 150                 155                 160


Trp Met Asn Asn Gln Phe Gly Gln Ser Leu Glu Leu Asp Gly Ile Ile
                165                 170                 175


Tyr Leu Gln Ala Thr Pro Glu Thr Cys Leu His Arg Ile Tyr Leu Arg
                180                 185                 190
```

```
Gly Arg Asn Glu Glu Gln Gly Ile Pro Leu Glu Tyr Leu Glu Lys Leu
        195                 200             205

His Tyr Lys His Glu Ser Trp Leu Leu His Arg Thr Leu Lys Thr Asn
        210                 215             220

Phe Asp Tyr Leu Gln Glu Val Pro Ile Leu Thr Leu Asp Val Asn Glu
225                 230             235                 240

Asp Phe Lys Asp Lys Tyr Glu Ser Leu Val Glu Lys Val Lys Glu Phe
                245             250                 255

Leu Ser Thr Leu
            260
```